# EUROPEAN PATENT APPLICATION

(11) **EP 2 149 373 A1**
(43) Date of publication of application: **03.02.2010**
(21) Application number: 08380239.7
(22) Date of filing: 01.08.2008
(51) Int. Cl.: A61K 31/495, A61K 31/496, A61K 31/506, A61K 31/515, A61P 9/00, A61P 13/00, A61P 25/00

(54) **5HT7 receptor ligands and compositions comprising the same**

(71) Applicant: Laboratorios Del. Dr. Esteve, S.A., 08041 Barcelona (ES)
(72) Inventor: Pascual-Ramon, Rosalia, 08173 Sant Cugat del Valles (Barcelona) (ES); Buschmann, Helmut H., 08960 Sant just Desvern (Barcelona) (ES)
(74) Representative: Carpintero Lopez, Francisco

(57) **Abstract**

The present invention relates to compounds having pharmacological activity towards the 5-HT₇ receptor, especially as agonists, identified by using a pharmacophore and a descriptor's profile filter as well as to pharmaceutical compositions comprising them. These compounds are useful in therapy in particular for the treatment and or prophylaxis of a disease in which 5-HT₇ is involved, such as CNS disorders.

## Description

### FIELD OF THE INVENTION

The present invention relates to compounds having pharmacological activity towards the 5-HT₇ receptor, especially as agonists, identified by using a pharmacophore and a descriptor's profile filter as well as to pharmaceutical compositions comprising them. These compounds are useful in therapy in particular for the treatment and or prophylaxis of a disease in which 5-HT₇ is involved, such as CNS disorders.

### BACKGROUND OF THE INVENTION

The search for new therapeutic agents has been greatly aided in recent years by better understanding of the structure of proteins and other biomolecules associated with target diseases. One important class of proteins that has been the subject of extensive study is the family of 5-hydroxytryptamine (serotonin, 5-HT) receptors. The 5-H₇ receptor discovered in 1993 belongs to this family and has attracted great interest as a valuable new drug target (Terrón, J.A. Idrugs, 1998, vol. 1, no. 3, pages 302-310: "The 5HT7 receptor: A target for novel therapeutic avenues?").

5-HT₇ receptors have been cloned from rat, mouse, guinea pig and human cDNA and exhibit a high degree of interspecies homology (approx. 95%), but it is unique in that it has a low sequence homology with other 5-HT receptors (less than 40%). Its expression pattern, in particular structures of the central nervous system (CNS) (highest in hypothalamus in particular suprachiasmatic nuclei and thalamus) and other peripheral tissues (spleen, kidney, intestinal, heart and coronary arthery), implicates the 5-HT₇ receptor in a variety of functions and pathologies. This idea is reinforced by the fact that several therapeutic agents, such as tricyclic antidepressants, typical and atypical antipsychotics and some 5-HT₂ receptor antagonists, display moderate to high affinity for both recombinant and functional 5-HT₇ receptors.

Functionally, the 5-HT₇ receptor has been implicated in regulation of circadian rhythms in mammals (Lovenberg, T.W. et al. Neuron, 1993, 11:449-458 "A novel adenylyl cyclase-activating serotonin receptor (5-HT7) implicated in the regulation of circadian rhythms"). It is known that disruption of circadian rhythms is related to a number of CNS disorders including depression, seasonal affective disorder, sleep disorders, shift worker syndrome and jet lag among others.

Distribution and early pharmacological data also suggest that the 5-HT₇ receptor is involved in the vasodilatation of blood vessels. This has been demonstrated *in vivo* (Terrón, J.A., Br J Pharmacol, 1997, 121:563-571 "Role of 5-HT7 receptors in the long lasting hypotensive response induced by 5-hydroxytryptamine in the rat"). Thus selective 5-HT₇ receptor agonists have a potential as novel hypertensive agents.

The 5-HT₇ receptor has also been related with the pathophysiology of migraine through smooth muscle relaxation of cerebral vessels (Schoeffter, P. et al., 1996, Br J Pharmacol, 117:993-994; Terrón, J.A., 2002, Eur. J. Pharmacol., 439:1-11 "Is the 5-HT7 receptor involved in the pathogenesis and prophylactic treatment of migraine?"). In a similar manner, involvement of 5-HT₇ in intestinal and colon tissue smooth muscle relaxation makes this receptor a target for the treatment of irritable bowel syndrome (De Ponti, F. et al. , 2001, Drugs, 61:317-332 "Irritable bowel syndrome. New agents targeting serotonin receptor subtypes"). Recently, it has also been related to urinary incontinence (British J. of Pharmacology, Sept. 2003, 140(1) 53-60: "Evidence for the involvement of central 5HT-7 receptors in the micurition reflex in anaeshetized female rats").

In view of the potential therapeutic applications of agonists or antagonists of the 5HT₇ receptor, a great effort has been directed to find selective ligands. Despite intense research efforts in this area, very few compounds with selective 5-HT₇ activity have been reported (Wesolowska, A., Polish J. Pharmacol., 2002, 54: 327-341, "In the search for selective ligands of 5-HT5, 5-HT6 and 5-HT7 serotonin receptors*"*).

WO 97/48681 discloses sulfonamide derivatives, which are 5-HT₇ receptor antagonists, for the treatment of CNS disorders.

Also, WO 97/29097 and WO9729097 describe sulfonamide derivatives for the treatment of disorders in which antagonism of the 5-HT₇ receptor is beneficial.

WO 03/048118 and WO 00/00472 describe different groups of 5HT₇ receptor antagonists.

WO99/24022 discloses tetrahydroisoquinoline derivatives for use against CNS disorders and binding to serotonin receptors, in particular 5-HT₇.

Nevertheless, there is still a need to find compounds that have pharmacological activity towards the receptor 5-HT₇, being both effective and selective.

A common approach for the discovery of new compounds with activity or affinity towards a certain therapeutic target is the development of pharmacophores. The concept of a 'pharmacophore' is not recent. It was first introduced by Paul Ehrlich in 1909 as "a molecular framework that carries (phoros) the essential features responsible for a drug's (pharmacon's) biological activity" [Über den jetzigen stand der chemotherapie. Chem. Ber. 42, 17]. This definition was further updated in 1977 by Peter Gund to "a set of structural features in a molecule that is recognized at a receptor site and is responsible for that molecule's biological activity" [Three-dimensional pharmacophoric pattern searching. Prog. Mol. Subcell. Biol. 5, 117 143]. More recently, the official IUPAC recommendation from 1997 has summarized the concept as follows: "A pharmacophore is the ensemble of steric and electronic features that is necessary to ensure the optimal supramolecular interactions with a specific biological target and to trigger (or block) its biological response" [Wermuth, C.-G. et al. (1998) Glossary of terms used in medicinal chemistry (IUPAC Recommendations 1998). Pure Appl. Chem. 70, 1129-1143]. Thus, a pharmacophore does not represent a real molecule or a real association of functional groups, but a purely abstract concept that accounts for the common molecular interaction capacities of a group of compounds towards their target structure. The pharmacophore can be considered as the largest common denominator shared by a set of active molecules. Pharmacophore are normally defined by pharmacophoric descriptors which include H-bonding, hydrophobic and electrostatic interaction sites, defined by atoms, ring centers and virtual points.

Up to now, several computational models of 5HT₇ receptor ligands have been described. Lopez Rodriguez et al. developed a pharmacophore model based on 30 known antagonist of 5HT₇ receptors [First pharmacophoric hypothesis for 5Ht7 antagonism (2000) bioorganic&medicinal chemistry letters 10(10): 1097-1100] which was later optimized with the incorporation of new ligands [Optimization of the pharmacophore model for 5-HT7R antagonism. Design and synthesis of new naphtolactam and naphtosultamderivatives (2003) Journal of Medicinal Chemistry 46(26):5638-5650].

Other pharmacophore models of 5HT₇ receptor antagonism were taught by Lepailleur A. et al [Molecular design based on 3D pharmacophores. Applications to 5-HT7 receptors (2004)Journal of chemical information and computer sciences 44(3):1148-1152] and Kolaczowski M. et al [Receptor-based pharmacophores for serotonin 5-HT7R antagonists-implications to selectivity (2006) Journal of Medicinal Chemistry 49(23):6732-6741].

Also pharmacophoric models of 5HT₇ receptor agonism have been disclosed. Vermeulen E.S. et al developed a pharmocophore of inverse agonism based on 22 inverse agonists [Novel 5-HT7 receptor inverse agonists. Synthesis and molecular modelling of arylpiperazine and 1,2,3,4-tetrahydroisoquinoline-based arylsulfonamides (2004) Journal of medicinal Chemistry 47(22): 5451-5466] as well as two models of 5HT₇ receptor agonism based on 20 diverse agonists *[*Characterization of the 5-HT7 Receptor. Determination of the pharmacophore for 5-HT7 receptor agonism and CoMFA-based modelling of the agonist binding site (2003) Journal of Medicinal Chemistry 46(25):5365-5374].

Now the authors of the present invention based on a novel pharmacophore model have identified several compounds which are particularly selective ligands of the 5HT₇ receptors. All this compounds share some common features which were useful to define a general formula I encompassing all of them.

### OBJECT OF THE INVENTION

It is an object of the present invention the use of compounds of general formula I: 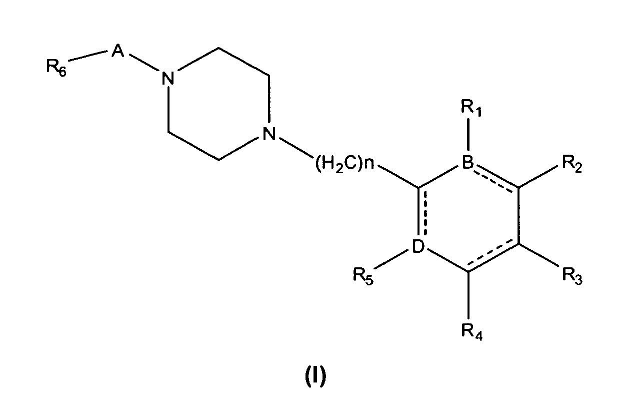 for the prophylaxis and treatment of diseases mediated or related to 5HT₇ receptors, that is, diseases caused by failures in central and peripheral serotonin-controlling functions, such as pain, sleep disorder, shift worker syndrome, jet lag, depression, seasonal affective disorder, migraine, anxiety, psychosis, schizophrenia, cognition and memory disorders, neuronal degeneration resulting from ischemic events, cardiovascular diseases such as hypertension, irritable bowel syndrome, inflammatory bowel disease, spastic colon or urinary incontinence.

The compounds encompassed by general formula I were identified by a method for screening ligands of 5HT₇ receptor, more specifically agonist of 5HT₇ receptors. The method comprises providing pharmacophore features and distances between features as described by a 5HT₇ receptor ligand pharmacophore as input to a 3-dimensional database; resultant matches filtered by a descriptor's profile (property profile) and selecting resultant structures for experimental screening by scaffold diversity. The screening is thus based on a pharmacophore model of agonism developed by the inventors. The screening may be carried out over a known library of compounds or conversely the pharmacophore model may be used to evaluate de novo designed compounds.

It is another object of the invention, a pharmaceutical composition comprising at least one compound of general formula I or a pharmaceutically acceptable salt, isomer, prodrug or solvate thereof, and at least a pharmaceutically acceptable carrier, adjuvant, additive or vehicle.

### BRIEF DESCRIPTION OF THE FIGURES

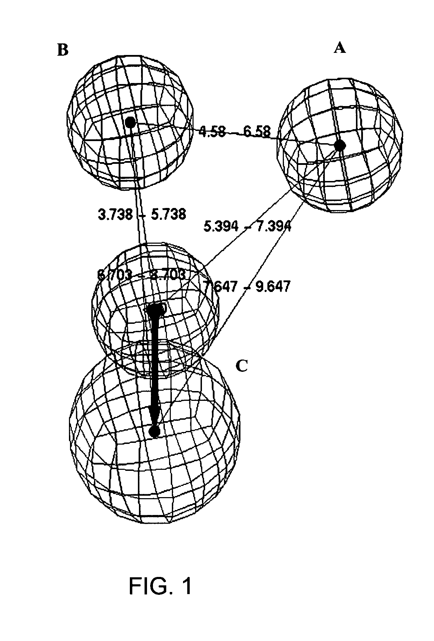

**Figure 1****:** represents the 3D pharmacophore model of the invention.

### DESCRIPTION OF THE INVENTION

In a first aspect, the invention is related to the use of a compound of general formula (I): 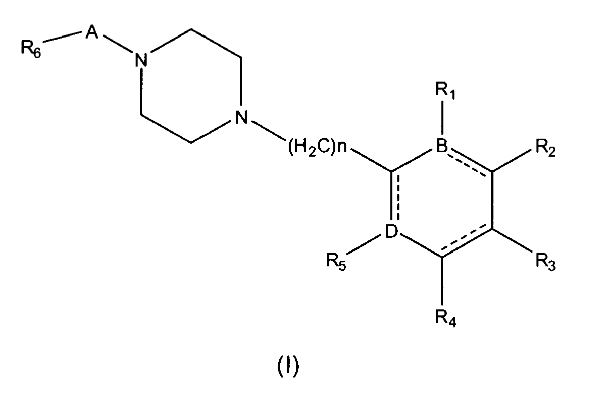 wherein
the dotted lines represent optional double bonds,
R₁, R₂, R₃, R₄, and R₅ can be selected independently from hydrogen, saturated or unsaturated, linear or branched unsubstituted or at least mono-substituted C₁₋₅ alkyl radical, -CF₃, halogen, nitro, -NR₉R₁₀ and -OR₉
or R₁ and R₂ form together with the benzene ring an indole system substituted in the position 4,
or R₃ and R₄ form together with the cyclohexane ring a 1,2,3,4-tetrahydronaphtalene system,
A can be a -CH₂- or a -C(O)-,
B and D represent C or N atoms n represents 0 or 1
R₆ can be selected from: 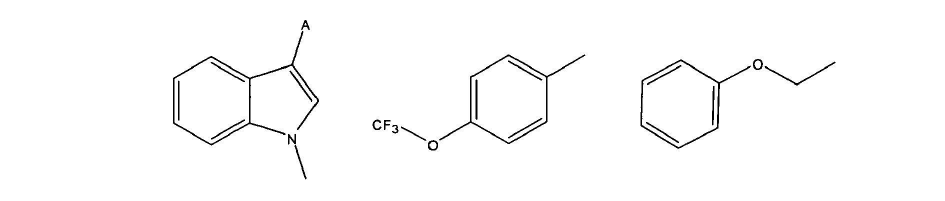 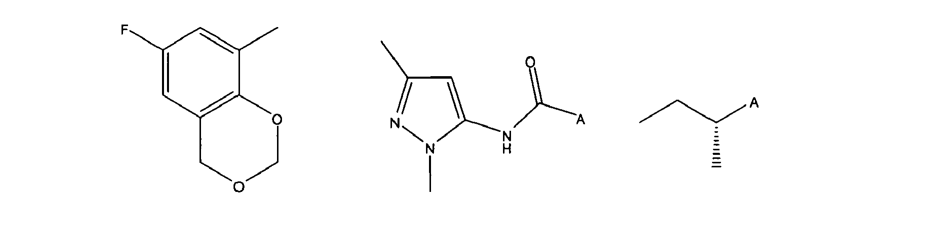 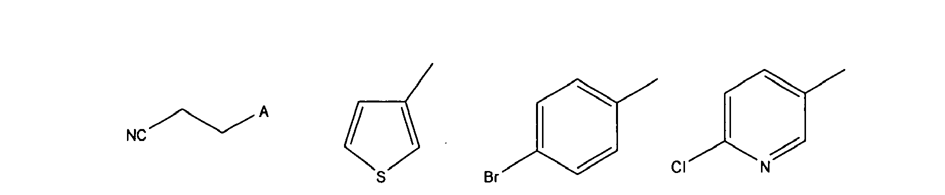 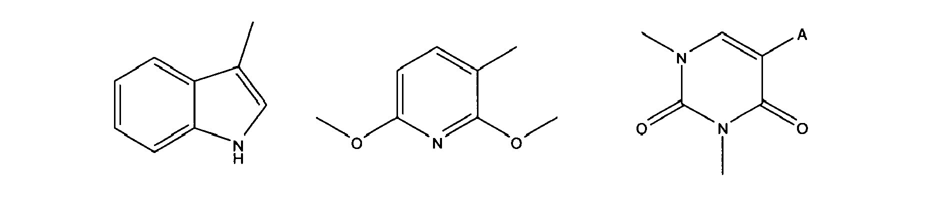 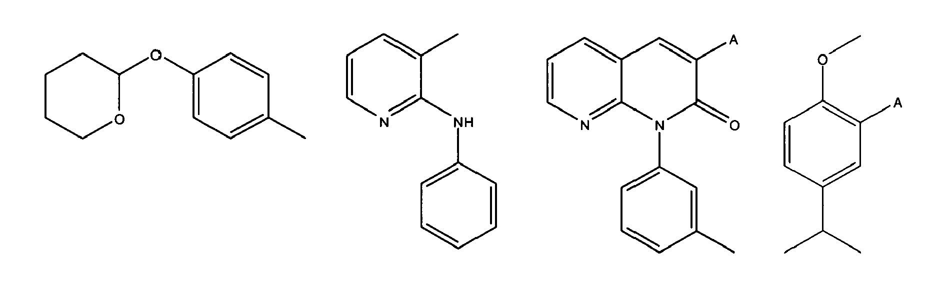 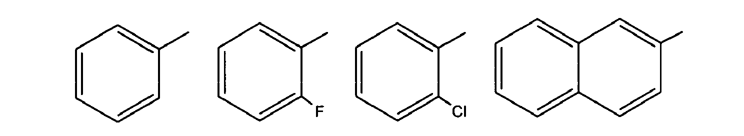 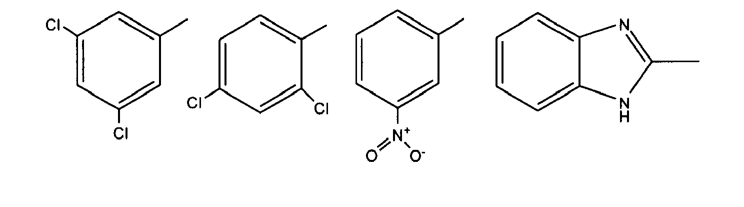 R₉ and R₁₀ can be selected independently from hydrogen or saturated or unsaturated, linear or branched unsubstituted or at least mono-substituted C₁₋₅ alkyl radical
optionally in form of one of its stereoisomers, preferably enantiomers or diasteromers, a racemate or in form of a mixture of at least two of its stereoisomers, preferably enantiomers and/or diasteromers, in any mixing ratio, or a salt thereof, or a corresponding solvate thereof for the manufacture of a medicament for the treatment of a 5-HT₇ mediated disease or condition.

In a particular and preferred embodiment the compounds of the invention are defined by a general formula (Ia): 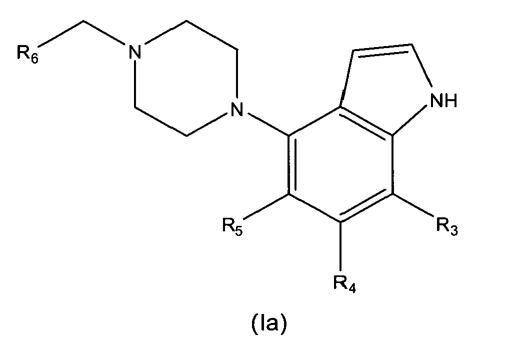 wherein R₃, R₄, R₅ and R₆ have the same meaning as before,
optionally in form of one of its stereoisomers, preferably enantiomers or diasteromers, a racemate or in form of a mixture of at least two of its stereoisomers, preferably enantiomers and/or diasteromers, in any mixing ratio, or a salt thereof, or a corresponding solvate thereof.

The compounds encompassed by general formula I and (Ia) were identified by a method for screening of compounds having 5-HT₇ receptor agonist activity, the method comprising:
a) providing 5HT₇ ligand pharmacophore features and distances between said features as input to a 3-dimensional database;
b) filtering resultant matches by a descriptor's profile and
c) selecting resultant structures for experimental screening by scaffold diversity

The specific 5HT₇ ligand pharmacophore features and distances between them of point a) are represented in figure 1 and comprise:
i) a positive ionizable group A, preferably a basic nitrogen, either aliphatic or aromatic.
ii) An aromatic moiety B separated from A by from 4.6 to 6.6 angstroms.
iii) A hydrogen bond accepting group, preferably an oxygen, sulfur or non-basic nitrogen, with a lone pair and charge less than or equal to zero, this group being separated from A and B by from 5.4 to 7.4 angstroms and by from 3.7 to 5.7 angstroms respectively and the directionality of the hydrogen bond being defined in the model by a projection point that stands for the donor.

The pharmacophore described herein was generated based on the shared features of a set of 8 compounds reported as 5HT₇ agonists. The 8 compounds were chosen because of their nanomolar affinity, low flexibility and structural diversity. Table 1 shows the structure of the 8 compounds used to build the pharmacophore model of the invention together with a reference where they were previously disclosed.

**Table 1: compounds used for the generation of the pharmacophore**

| **STRUCTURE** | **Reference** | **STRUCTURE** | **Reference** |
|---|---|---|---|
| 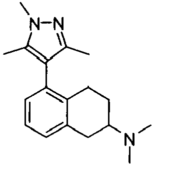 | Tocris Cookson (Pharmacologi cal Tool) | 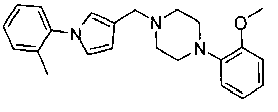 | Bioorg. Med. Chem. Lett. 2005, 15, 3753 |
| 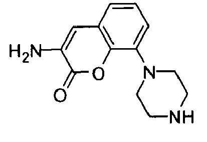 | US 0215551, US 0215567 | 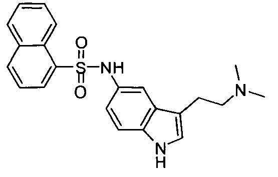 | Internal reference (ESTEVE) |
| 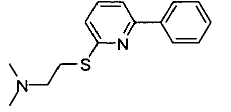 | Bioorg. Med. Chem. Lett. 2004, 14, 677-680 | 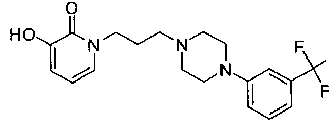 | EP 00998923 |
| 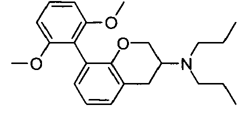 | J. Med. Chem. 2004,47, 3927-3930 | 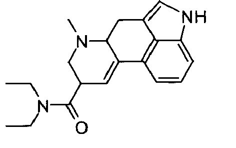 | J. Med. Chem. 2003,46, 5365-5374 |

As a guidance for the correct alignment and selection of shared interaction features of the reference compounds, a 5HT₇ receptor homology model (data not shown) as well as knowledge about the binding region of class A GPCR receptors (VAN RHEE, A.M. & JACOBSEN, K.A. (1996) Molecular architecture of G-protein coupled receptors. Drug Dev. Res. 37: 1-38; Strader et al. J. Biol. Chem. 1988 263: 10267-10271; Liapakis et al. Biol. Chem. 2000 275: 37779-37788) were used. This information gave clues about which amino acids and hence what kind of interactions and geometrical arrangements of them were possible.

The pharmacophore was generated with the Discovery studio 2.0 software (Accelrys Inc., San Diego, CA).

Once the pharmacophore was generated, the pharmacophore features and distances between said features were used as input to a 3-dimensional database where each compound is stored as an ensemble of representative conformers within about 20 kcal/mol above the calculated global minimum (See Grigorov, M, et al. (1995) J. Chem.Inf. Comput. Sci. 35: 285-304). Although Discovery Studio 2.0 software (Accelrys Inc., San Diego, CA) was preferred for pharmacophore generation and library screening, other methods known in the art such as those described in PHARMACOPHORE PERCEPTION, DEVELOPMENT, AND USE IN DRUG DESIGN (2000) Ed. Osman F. Gunner, International University Line, La Jolla, CA, might be used.

As many compounds might have the feature disposition given by the pharmacophore but with more functionality that does not have a proper interaction with the receptor, thus hindering binding, additional filters were used in the method of screening.

Step b) of the method comprised subjecting the resultant matches to a property profile filter (descriptor's profile filter) based on properties of the reference agonists with an added tolerance which was established and chosen as a second step due to its efficiency and good performance on test libraries.

The profile filter applied comprised the following descriptors and value ranges:
- Molecular Weight < 400
- Number of Rings in the molecule < 6
- Number of carbon atoms: a_C < 30
- Number of iodine atoms: a_I = 0
- Number of oxygen atoms: a_O < 6
- Number of nitrogen atoms: a_N < 6
- Number of halogen atoms: a_Hal < 7
- Number of heavy atoms different than C: a_noC < 11
- Number of oxygen and nitrogen together: a_O+a_N < 7
- Number of aromatic atoms: a_aro < 20
- Number of aromatic bonds: 0 < b_aro < 22
- Number of ratable single bonds: b_1rot < 10
- Number of triple bonds: b_triple < 2
- Formal charge of the molecule in neutral form: Fcharge noprot = -1, 0, 1 or 2.
- Formal charge of the molecule in the ionization state predicted at pH 7.4: Fcharge_pH= 0, 1 or 2.
- Number of acceptor atoms of the molecule in neutral form: 0 < a_acc_noprot < 4
- Number of acceptor atoms of the molecule in the ionization state predicted at pH 7.4: a_acc_pH < 3
- Number of donor atoms of the molecule in neutral form: a_don_noprot < 3
- Number of donor atoms of the molecule in the ionization state predicted at pH 7.4: a_don_pH < 2
- Difference in number of acceptor atoms between the pH adjusted and the neutral fo rm: a_acc_noprot - a_acc_pH < 4
- Diameter of the molecule defined as the largest vertex eccentricity of the molecular graph: 4 < diameter < 17
- Radius of the molecule defined as the shortest vertex eccentricity of the molecular graph: 2 < radius < 9
- Petitjean: value of (diameter - radius) / diameter: Petitjean < 0.5
- Van der Waals volume calculated using a connection table approximation: vdw_vol < 600
- Polar surface area calculated using group contributions to approximate the polar surface area from connection table information only. TPSA <= 80 [J. Med. Chem. 43, 3714-3717 (2000)]
- Kier molecular flexibility index: Kierflex < 6 [Hall, L.H., Kier, L.B.; Reviews of Computational Chemistry. 2, (1991)]
- Wiener path number: WeinerPath < 2500 [Balaban, A.T.; Theoretica Chimica Acta. 53, 355-375(1979)]
- Atomic connectivity index order 0: Chi0 < 20 [Hall, L.H., Kier, L.B.; Reviews of Computational Chemistry. 2, (1991)]
- Predicted octanol/water partition coefficient: logP(o/w) < 6.5

The profile filtering was performed within the MOE software package, although those properties could have been calculated in a variety of software packages. Next, step c) of the method was carried out. Step c) was aimed to enrich the hit ratio of the final selection, that is, to enrich the number of compounds that were going to give positive results in the experimental binding assay. To this end a scaffold diversity selection was performed.

This task was also carried out within the MOE software package using the program scripting facilities, where a scaffold is chemoinformatically understood as all atoms and bonds forming ring systems and connecting ring systems within a structure. In all cases the simplest structure was selected for experimental evaluation. Of course other software packages could have been used for this task.

Some of the compounds identified as 5HT₇ ligands with good affinity by the method above described are specified in the following list:
a. 3-(2-aminoethyl)-N,N-dimethyl-1 H-indole-5-sulfonamide
b. 1-((6-chloropyridin-3-yl)methyl)-4-(2-isopropoxyphenyl)piperazine
c. 3-((4-(2-ethoxyphenyl)piperazin-1-yl)methyl)-1H-indole
d. 3-(4-(2-methoxyphenyl)piperazin-1-yl)-1-phenylpropan-1-ol
e. 1-(4-(1H-imidazol-1-yl)benzyl)-4-(2-isopropoxyphenyl)piperazine
f. N-(2,3-dihydro-1H-inden-5-yl)-3-(4-(2-methoxyphenyl)piperazin-1-yl)propanamide
g. 4-(4-(5-isopropyl-2-methoxybenzyl)piperazin-1-yl)-1H-indole
h. 1-(1-(3,4-difluorobenzyl)piperidin-4-yl)-1H-benzo[d]imidazol-2(3H)-one
i. 1-(1-(2-phenoxyethyl)piperidin-4-yl)-1H-benzo[d]imidazol-2(3H)-one
j. 1-(1-(3-phenoxypropyl)-1,2,3,6-tetrahydropyridin-4-yl)-1H-benzo[d]imidazol-2(3H)-one
k. 1-(1-(2-phenoxyethyl)-1,2,3,6-tetrahydropyridin-4-yl)-1H-benzo[d]imidazol-2(3H)-one
I. quinuclidin-3-yl(thiophen-2-yl)methanone

Based on scaffold diversity, availability and screening data (EC50: 6.62E-09) the compound (g) was chosen as agonist reference hit.

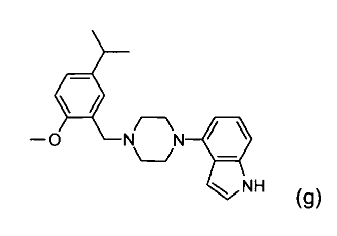

Then a further search for analogue compounds thereof was done in the internal library leading to the following compound with 5HT₇ affinity:
**[1]** 1-methyl-3-((4-phenylpiperazin-1-yl)methyl)-1H-indole
**[2]** 3-(4-(4-(trifluoromethoxy)benzyl)piperazin-1-yl)phenol
**[3]** 4-(4-(2-phenoxyethyl)piperazin-1-yl)-1H-indole
**[4]** 1-((6-fluoro-4H-benzo[d][1,3]dioxin-8-yl)methyl)-4-(1,2,3,4-tetrahydronaphthalen-2-yl)piperazine
**[5]** 2-(4-(1H-indol-4-yl)piperazin-1-yl)-N-(1,3-dimethyl-1H-pyrazol-5-yl)acetamide
**[6]** (S)-1-(4-(5-isopropyl-2-methoxybenzyl)piperazin-1-yl)-2-methylbutan-1-one
**[7]** 4-(4-(1H-indol-4-yl)piperazin-1-yl)butanenitrile
**[8]** (4-(5-isopropyl-2-methoxybenzyl)piperazin-1-yl)(thiophen-3-yl)methanone
**[9]** (4-bromophenyl)(4-(5-isopropyl-2- methoxybenzyl)piperazin-1-yl)methanone
**[10]** 1-((6-chloropyridin-3-yl)methyl)-4-(2,4- dimethylphenyl)piperazine
**[11]** 2-(1H-indol-3-yl)-1-(4-(5-isopropyl-2- methoxybenzyl)piperazin-1-yl)ethanone
**[12]** 1-(3,4-dimethoxyphenyl)-4-((2,6-dimethoxypyridin-3-yl)methyl)piperazine
**[13]** 1-(2,4-dimethylphenyl)-4-(4-(tetrahydro-2H-pyran-2-yloxy)benzyl)piperazine
**[14]** N-phenyl-3-((4-(3-(trifluoromethyl)phenyl)piperazin-1-yl)methyl)pyridin-2-amine
**[15]** 1-((2,6-dimethoxypyridin-3-yl)methyl)-4-(2- isopropoxyphenyl)piperazine
**[16]** 1-((2,6-dimethoxypyridin-3-yl)methyl)-4-(2,4- dimethylphenyl)piperazine
**[17]** 1,3-dimethyl-5-((4-(3-(trifluoromethyl)phenyl)piperazin-1-yl)methyl)pyrimidine-2,4(1 H,3H)-dione
**[18]** 1-(4-chlorophenyl)-4-((2,6-dimethoxypyridin-3-yl)methyl)piperazine
**[19]** 4-(4-(4-(tetrahydro-2H-pyran-2- yloxy)benzyl)piperazin-1-yl)-1H-indole
**[20]** 3-((4-(1H-indol-4-yl)piperazin-1-yl)methyl)-1-m-tolyl-1,8-naphthyridin-2(1H)-one
**[21]** 1-((2,6-dimethoxypyridin-3-yl)methyl)-4-(3-(trifluoromethyl)phenyl)piperazine
**[22]** 4-(4-((2,6-dimethoxypyridin-3-yl)methyl)piperazin-1- yl)-1H-indole
**[23]** 1-(4-chlorophenyl)-4-(5-isopropyl-2- methoxybenzyl)piperazine
**[24]** 1-(2,4-dimethylphenyl)-4-(5-isopropyl-2- methoxybenzyl)piperazine
**[25]** 4-(4-((6-chloropyridin-3-yl)methyl)piperazin-1-yl)- 1 H-indole
**[26]** 3-((4-(4-nitrophenyl)piperazin-1-yl)methyl)-N- phenylpyridin-2-amine
**[27]** 2-(4-(5-isopropyl-2-methoxybenzyl)piperazin-1-yl)pyrimidine
**[28]** 2-((4-(1H-indol-4-yl)piperazin-1-yl)methyl)-1H- benzo[d]imidazole
**[29]** 4-(4-(5-isopropyl-2-methoxybenzyl)piperazin-1-yl)- 1 H-indole
**[30]** 4-(4-benzylpiperazin-1-yl)-1H-indole
**[31]** 4-(4-(2-fluorobenzyl)piperazin-1-yl)-1H-indole
**[32]** 4-(4-(naphthalen-2-ylmethyl)piperazin-1-yl)-1H-indole
**[33]** 4-(4-(2-chlorobenzyl)piperazin-1-yl)-1H-indole
**[34]** 4-(4-(3,4-dichlorobenzyl)piperazin-1-yl)-1H-indole
**[35]** 4-(4-(2,4-dichlorobenzyl)piperazin-1-yl)-1H-indole
**[36]** 4-(4-(3-nitrobenzyl)piperazin-1-yl)-1H-indole

All of these compounds are encompassed within general formula I. The most preferred compounds of the invention are:
**[4]** 1-((6-fluoro-4H-benzo[d][1,3]dioxin-8-yl)methyl)-4-(1,2,3,4-tetrahydronaphthalen-2-yl)piperazine
**[19]** 4-(4-(4-(tetrahydro-2H-pyran-2- yloxy)benzyl)piperazin-1-yl)-1H-indole
**[23]** 1-(4-chlorophenyl)-4-(5-isopropyl-2- methoxybenzyl)piperazine
**[24]** 1-(2,4-dimethylphenyl)-4-(5-isopropyl-2- methoxybenzyl)piperazine
**[25]** 4-(4-((6-chloropyridin-3-yl)methyl)piperazin-1-yl)- 1 H-indole

These compounds have been identified and distinguished as 5HT₇ agonist by the method of screening described herein. Their affinity for 5-HT7 receptor is expressed by their %-Inhib. (10⁻⁶ M) and IC50 as shown in table 2 (see example 1).

As the compounds of general formula I, are potent 5HT₇ agonists they are useful to treat 5HT₇ mediated diseases or conditions such as pain, sleep disorder, shift worker syndrome, jet lag, depression, seasonal affective disorder, migraine, anxiety, psychosis, schizophrenia, cognition and memory disorders, neuronal degeneration resulting from ischemic events, cardiovascular diseases such as hypertension, irritable bowel syndrome, inflammatory bowel disease, spastic colon or urinary incontinence.

Another aspect of the invention is related to a pharmaceutical composition comprising at least a compound of general formula I or a pharmaceutically acceptable salt, isomer, prodrug or solvate thereof, and a pharmaceutically acceptable carrier, adjuvant, additive or vehicle.

The auxiliary materials or additives can be selected among carriers, excipients, support materials, lubricants, fillers, solvents, diluents, colorants, flavour conditioners such as sugars, antioxidants and/or agglutinants. In the case of suppositories, this may imply waxes or fatty acid esters or preservatives, emulsifiers and/or carriers for parenteral application. The selection of these auxiliary materials and/or additives and the amounts to be used will depend on the form of application of the pharmaceutical composition.

The pharmaceutical composition in accordance with the invention can be adapted to any form of administration, be it orally or parenterally, for example pulmonarily, nasally, rectally and/or intravenously. Therefore, the formulation in accordance with the invention may be adapted for topical or systemic application, particularly for dermal, subcutaneous, intramuscular, intra-articular, intraperitoneal, pulmonary, buccal, sublingual, nasal, percutaneous, vaginal, oral or parenteral application.

Suitable preparations for oral applications are pills, chewing gums, capsules, granules, drops or syrups.

Suitable preparations for parenteral applications are solutions, suspensions, reconstitutable dry preparations or sprays.

The compounds of the invention may be administered as deposits in dissolved form or in patches, for percutaneous application.

Skin applications include ointments, gels, creams, lotions, suspensions or emulsions.
The preferred form of rectal application is by means of suppositories.

The daily dosage for humans and animals may vary depending on factors that have their basis in the respective species or other factors, such as age, weight or degree of illness and so forth. The daily dosage for mammals including humans usally ranges from 1 milligram to 2000 milligram, preferably 1 to 1500 mg, more preferably 1 to 1000 mg of substance to be administered during one or several intakes.

### Example 1: identification of 5-HT₇ receptor agonists of the invention

A library of about 53500 compounds in multiconformations three-dimensional format was screened with the above described method.

After the inputting the pharmacophore features in the three dimensional database and screening with Discovery Studio 2.0 software (Accelrys Inc., San Diego, CA), 17170 compounds were found to match the pharmacophore query.

To further reduce the number of candidate compounds the descriptor's profile filter was run. The descriptor's profile applied as filter was identical to that previously disclosed in the description. This filter was performed with the MOE software package. The number of compounds was reduced from 17170 down to 3609 hit candidates by the property profile filter applied.

Next, 480 compounds were selected after the scaffold diversity selection with MOE software package and 461 of them were available for 5HT₇ binding experiments.

12 compounds showed good affinity for the receptor with even nanomolar values and displayed the desired 5HT₇ agonist functionality. From these 12 compounds 4-(4-(5-isopropyl-2-methoxybenzyl)piperazin-1-yl)-1H-indole (compound g) was chosen as reference hit to carry out a further search for analog compounds in the internal library.
Table 2 shows the compounds with 5HT7 affinity as agonist identified.

### Pharmacological Methods:

### Radioligand binding

Radioligand binding assays were performed using the Cloned Human Serotonin Receptor, Subtype 7 (h5HT₇), expressed in CHO cells, coated on Flashplate (Basic Flashplate Cat.: SMP200) from PerkinElmer (Cat.: 6120512). The protocol assay was essentially the recommended protocol in the Technical Data Sheet by PerkinEmer Life and Analytical Sciences. The Mass membrane protein/well was typically 12 µg and the Receptor/well was about 9-10 fmoles. The Flashplate were let equilibrate at room temperature for one hour before the addition of the components of the assay mixture. The binding buffer was: 50 mM Tris-HCl, pH 7.4, containing 10 mM MgCl₂, 0.5 mM EDTA and 0.5% BSA. The radioligand was [¹²⁵I]LSD at a final concentration of 0.82 nM. Nonspecific binding was determined with 50 µM of Clozapine. The assay volume was 25 µl. TopSeal-A were applied onto Flashplate microplates and they were incubated at room temperature for 240 minutes in darkness. The radioactivity were quantified by liquid scintillation spectrophotometry (Wallac 1450 Microbeta Trilux) with a count delay of 4 minutes prior to counting and a counting time of 30 seconds per well. Competition binding data were analyzed by using the LIGAND program (Munson and Rodbard, LIGAND: A versatile, computerized approach for characterization of ligand-binding systems. Anal. Biochem. 107: 220-239, 1980) and assays were performed in triplicate determinations for each point.

**Table 2: list of compounds identified as 5HT₇ agonists**

| **Ex** | **STRUCTURE** | **CHEMICAL NAME** | **%- Inhib. (10⁻⁶ M)** | **IC₅₀ (nM)** | **EC₅₀ (**µ**M) agonist** |
|---|---|---|---|---|---|
| **1** | 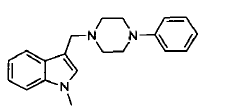 | 1-methyl-3-((4-phenylpiperazin-1-yl)methyl)-1H-indole | 22.3 | | |
| **2** | 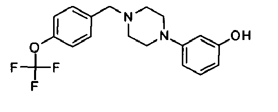 | 3-(4-(4-(trifluoromethoxy)benzyl) piperazin-1-yl)phenol | 15.1 | | |
| **3** | 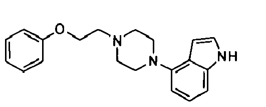 | 4-(4-(2-phenoxyethyl)piperazin-1-yl)-1H-indole | 53.7 | 1938 | |
| **4** | 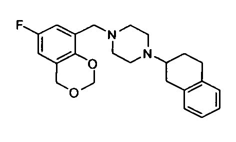 | 1-((6-fluoro-4H-benzo[d] [1,3]dioxin-8-yl)methyl)-4-(1,2,3,4-tetrahydronaphthalen-2-yl)piperazine | 49 | 2330 | |
| **5** | 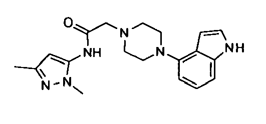 | 2-(4-(1H-indol-4-yl)piperazin-1-yl)-N-(1,3-dimethyl-1 H-pyrazol-5-yl)acetamide | 14.3 | | |
| **6** | 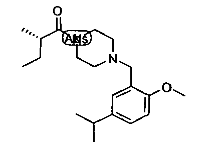 | (S)-1-(4-(5-isopropyl-2-methoxybenzyl) piperazin-1- yl)-2-methylbutan-1-one | 15 | | |
| **7** | 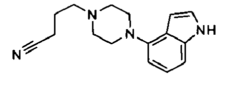 | 4-(4-(1H-indol-4-yl)piperazin-1-yl)butanenitrile | 49.6 | 712.6 | |
| **8** | 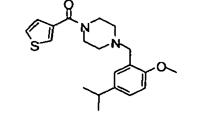 | (4-(5-isopropyl-2-methoxybenzyl)piperazin-1-yl)(thiophen-3-yl)methanone | 30.5 | | |
| **9** | 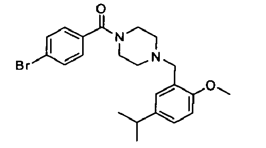 | (4-bromophenyl)(4-(5-isopropyl-2-methoxybenzyl)piperazin-1-yl)methanone | 68.1 | 1794 | |
| **10** | 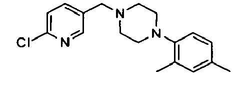 | 1-((6-chloropyridin-3-yl)methyl)-4-(2,4-dimethylphenyl)piperazine | 16.6 | | |
| **11** | 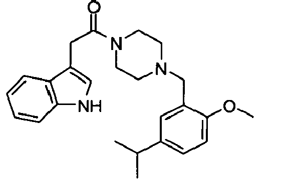 | 2-(1H-indol-3-yl)-1-(4-(5-isopropyl-2-methoxybenzyl)piperazin-1-yl)ethanone | 16.3 | | |
| **12** | 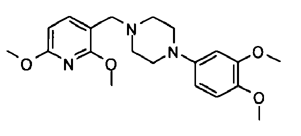 | 1-(3,4-dimethoxyphenyl)-4-((2,6-dimethoxypyridin-3-yl)methyl)piperazine | -0.3 | | |
| **13** | 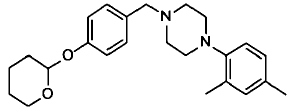 | 1-(2,4-dimethylphenyl)-4-(4-(tetrahydro-2H-pyran-2-yloxy)benzyl)piperazine | 12.1 | | |
| **14** | 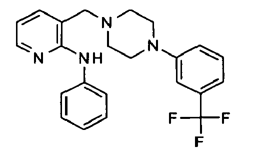 | N-phenyl-3-((4-(3-(trifluoromethyl)phenyl)pip erazin-1-yl)methyl)pyridin-2-amine | 19.5 | | |
| **15** | 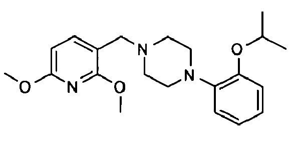 | 1-((2,6-dimethoxypyridin-3-yl)methyl)-4-(2-isopropoxyphenyl)piperazi ne | 92.2 | 82.2 | |
| **16** | 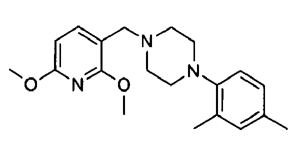 | 1-((2,6-dimethoxypyridin-3-yl)methyl)-4-(2,4-dimethylphenyl)piperazine | 8.8 | | |
| **17** | 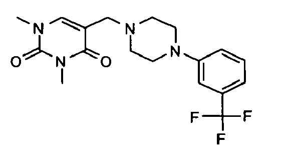 | 1,3-dimethyl-5-((4-(3-(trifluoromethyl)phenyl)pip erazin-1-yl)methyl)pyrimidine-2,4(1H,3H)-dione | 28.5 | | |
| **18** | 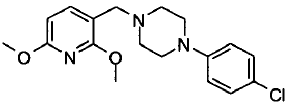 | 1-(4-chlorophenyl)-4-((2,6-dimethoxypyridin-3-yl)methyl)piperazine | 12.3 | | |
| **19** | 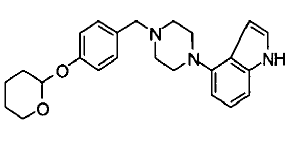 | 4-(4-(4-(tetrahydro-2H-pyran-2-yloxy)benzyl)piperazin-1-yl)-1H-indole | 59.5 | 1140.4 | |
| **20** | 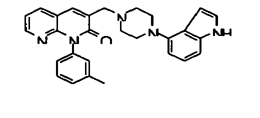 | 3-((4-(1H-indol-4-yl)piperazin-1-yl)methyl)-1-m-tolyl-1,8-naphthyridin-2(1 H)-one | 51.9 | 423.3 | 502 |
| **21** | 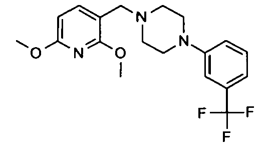 | 1-((2,6-dimethoxypyridin-3-yl)methyl)-4-(3-(trifluoromethyl)phenyl)pip erazine | 9.4 | | |
| **22** | 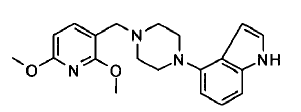 | 4-(4-((2,6-dimethoxypyridin-3-yl)methyl)piperazin-1- yl)-1 H-indole | 113.8 | 109 | 64.2 |
| **23** | 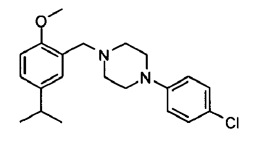 | 1-(4-chlorophenyl)-4-(5-isopropyl-2-methoxybenzyl)piperazine | 35.9 | | |
| **24** | 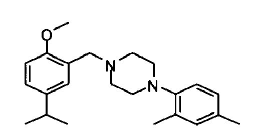 | 1-(2,4-dimethylphenyl)-4-(5-isopropyl-2-methoxybenzyl)piperazine | 41.6 | | |
| **25** | 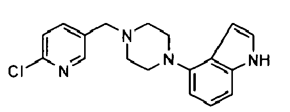 | 4-(4-((6-chloropyridin-3-yl)methyl)piperazin-1-yl)-1 H-indole | 66.8 | 328.4 | 789 |
| **26** | 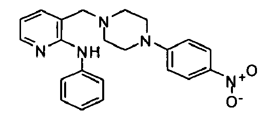 | 3-((4-(4-nitrophenyl)piperazin-1-yl)methyl)-N-phenylpyridin-2-amine | -4.6 | | |
| **27** | 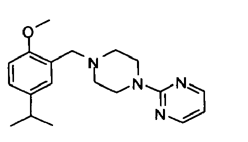 | 2-(4-(5-isopropyl-2-methoxybenzyl)piperazin-1-yl)pyrimidine | 48.9 | | |
| **28** | 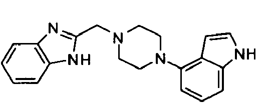 | 2-((4-(1H-indol-4-yl)piperazin-1-yl)methyl)-1H-benzo[d]imidazole | 28.8 | | 709 |
| **29** | 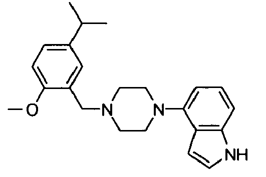 | 4-(4-(5-isopropyl-2-methoxybenzyl)piperazin-1-yl)- 1H-indole | 76.6 | 28.7 | 6.62 |
| **30** | 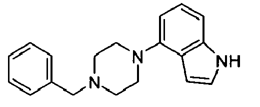 | 4-(4-benzylpiperazin-1-yl)-1H-indole | 83,1 | 338,73 | |
| **31** | 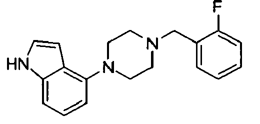 | 4-(4-(2-fluorobenzyl)piperazin-1-yl)-1H-indole | 82,6 | 459,62 | |
| **32** | 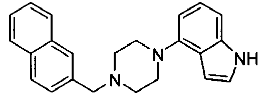 | 4-(4-(naphthalen-2-ylmethyl)piperazin-1-yl)-1H-indole | 78,4 | 760,24 | |
| **33** | 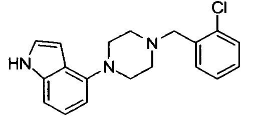 | 4-(4-(2-chlorobenzyl)piperazin-1-yl)-1H-indole | 78,2 | 222,93 | |
| **34** | 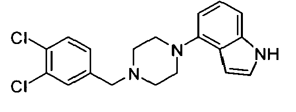 | 4-(4-(3,4-dichlorobenzyl)piperazin-1-yl)-1H-indole | 54,7 | 1243,4 4 | |
| **35** | 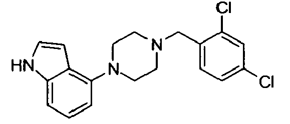 | 4-(4-(2,4-dichlorobenzyl)piperazin-1-yl)-1H-indole | 51,3 | | |
| **36** | 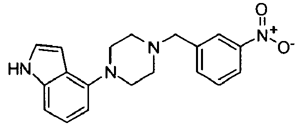 | 4-(4-(3-nitrobenzyl)piperazin-1-yl)-1H-indole | 41,5 | | |

All of these compounds were commercially available.

## Claims

1. Use of a compound of general formula (I): 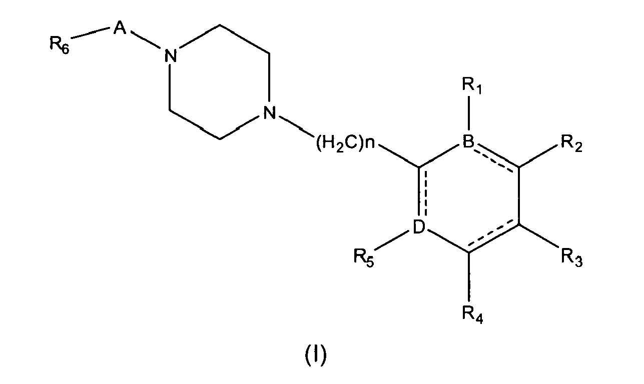 wherein
the dotted lines represent optional double bonds
R₁, R₂, R₃, R₄, and R₅ can be selected independently from hydrogen, saturated or unsaturated, linear or branched unsubstituted or at least mono-substituted C₁₋₅ alkyl radical, -CF₃, halogen, nitro, -NR₉R₁₀ and -OR₉
or R₁ and R₂ form together with the benzene ring an indole system substituted in the position 4,
or R₃ and R₄ form together with the cyclohexane ring a 1,2,3,4-tetrahydronaphtalene system,
A can be a -CH₂- or a -C(O)-,
B and D represent C or N atoms
n represents 0 or 1
R₆ can be selected from: 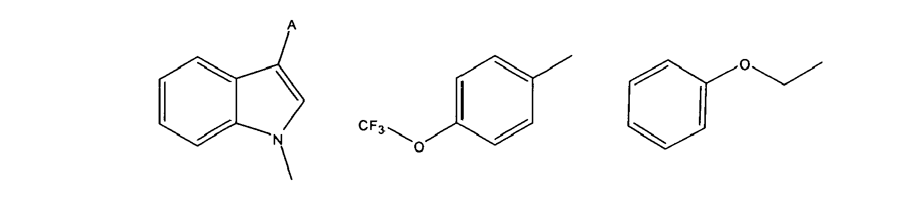 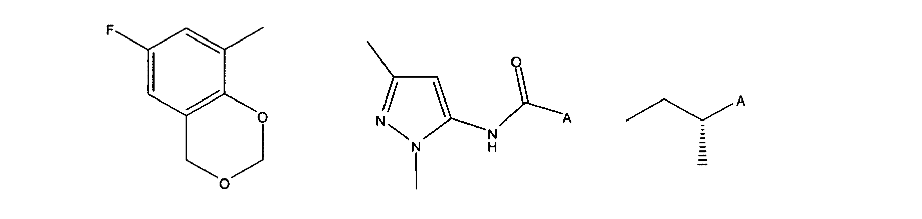 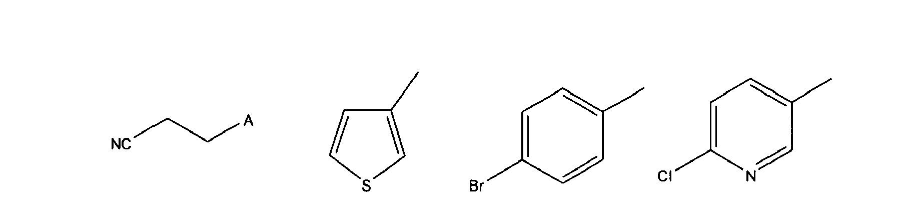 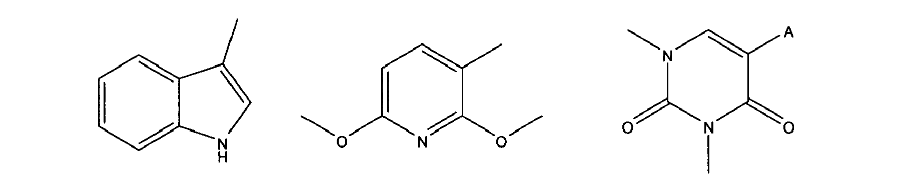 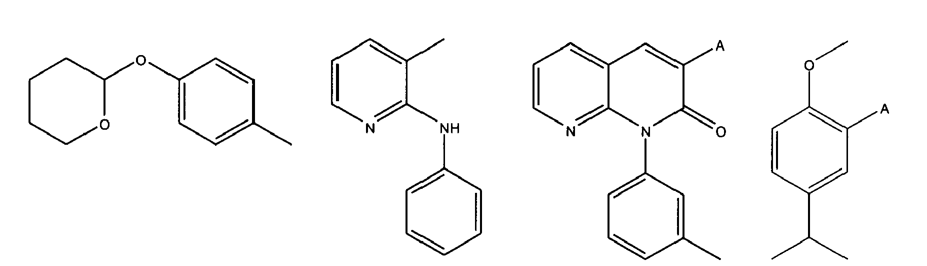 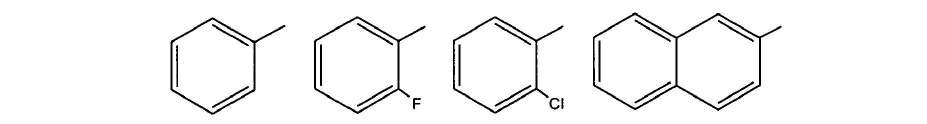 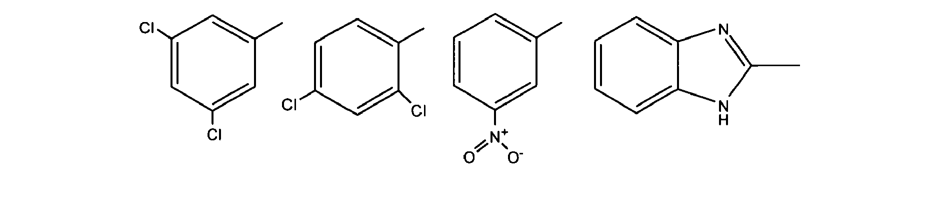 R₉ and R₁₀ can be selected independently from hydrogen or saturated or unsaturated, linear or branched unsubstituted or at least mono-substituted C₁₋₅ alkyl radical
optionally in form of one of its stereoisomers, preferably enantiomers or diasteromers, a racemate or in form of a mixture of at least two of its stereoisomers, preferably enantiomers and/or diasteromers, in any mixing ratio, or a salt thereof, or a corresponding solvate thereof for the manufacture of a medicament for the treatment of a 5-HT₇ mediated disease or condition.

2. Use of a compound of formula (Ia): 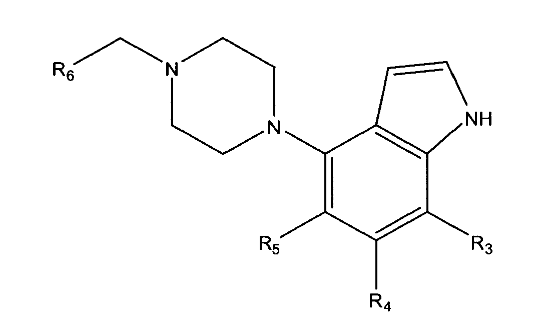 wherein R3, R4, R5 and R6 have the same meaning as in claim 1,
optionally in form of one of its stereoisomers, preferably enantiomers or diasteromers, a racemate or in form of a mixture of at least two of its stereoisomers, preferably enantiomers and/or diasteromers, in any mixing ratio, or a salt thereof, or a corresponding solvate thereof for the manufacture of a medicament for the treatment of a 5-HT₇ mediated disease or condition.

3. Use of a compound according to claim 1 selected from:
[1] 1-methyl-3-((4-phenylpiperazin-1-yl)methyl)-1H- indole
[2] 3-(4-(4-(trifluoromethoxy)benzyl)piperazin-1-yl)phenol
[3] 4-(4-(2-phenoxyethyl)piperazin-1-yl)-1H-indole
[4] 1-((6-fluoro-4H-benzo[d][1,3]dioxin-8-yl)methyl)-4-(1,2,3,4-tetrahydro naphthalen-2-yl)piperazine
[5] 2-(4-(1H-indol-4-yl)piperazin-1-yl)-N-(1,3-dimethyl-1H-pyrazol-5-yl)acetamide
[6] (S)-1-(4-(5-isopropyl-2-methoxybenzyl)piperazin-1-yl)-2-methylbutan-1-one
[7] 4-(4-(1H-indol-4-yl)piperazin-1-yl)butanenitrile
[8] (4-(5-isopropyl-2-methoxybenzyl)piperazin-1- yl)(thiophen-3-yl)methanone
[9] (4-bromophenyl)(4-(5-isopropyl-2-methoxybenzyl)piperazin-1-yl)methanone
[10] 1-((6-chloropyridin-3-yl)methyl)-4-(2,4-dimethylphenyl)piperazine
[11] 2-(1H-indol-3-yl)-1-(4-(5-isopropyl-2-methoxybenzyl)piperazin-1-yl)ethanone
[12] 1-(3,4-dimethoxyphenyl)-4-((2,6-dimethoxypyridin-3-yl)methyl)piperazine
[13] 1-(2,4-dimethylphenyl)-4-(4-(tetrahydro-2H-pyran-2-yloxy)benzyl)piperazine
[14]N-phenyl-3-((4-(3-(trifluoromethyl)phenyl)piperazin-1-I)methyl)pyridin-2-amine
[15] 1-((2,6-dimethoxypyridin-3-yl)methyl)-4-(2-isopropoxyphenyl)piperazine
[16] 1-((2,6-dimethoxypyridin-3-yl)methyl)-4-(2,4-dimethylphenyl)piperazine
[17] 1,3-dimethyl-5-((4-(3-(trifluoromethyl)phenyl)piperazin-1-yl)methyl) pyrimidine-2,4(1H,3H)-dione
[18] 1-(4-chlorophenyl)-4-((2,6-dimethoxypyridin-3-yl)methyl)piperazine
[19] 4-(4-(4-(tetrahydro-2H-pyran-2-yloxy)benzyl)piperazin-1-yl)-1H-indole
[20] 3-((4-(1H-indol-4-yl)piperazin-1-yl)methyl)-1-m-tolyl-1,8-naphthyridin-2(1 H)-one
[21] 1-((2,6-dimethoxypyridin-3-yl)methyl)-4-(3-(trifluoromethyl)phenyl) piperazine
[22] 4-(4-((2,6-dimethoxypyridin-3-yl)methyl)piperazin-1-yl)-1H-indole
[23] 1-(4-chlorophenyl)-4-(5-isopropyl-2- methoxybenzyl)piperazine
[24] 1-(2,4-dimethylphenyl)-4-(5-isopropyl-2- methoxybenzyl)piperazine
[25] 4-(4-((6-chloropyridin-3-yl)methyl)piperazin-1-yl)- 1H-indole
[26] 3-((4-(4-nitrophenyl)piperazin-1-yl)methyl)-N- phenylpyridin-2-amine
[27] 2-(4-(5-isopropyl-2-methoxybenzyl)piperazin-1- yl)pyrimidine
[28] 2-((4-(1H-indol-4-yl)piperazin-1-yl)methyl)-1H- benzo[d]imidazole
[29] 4-(4-(5-isopropyl-2-methoxybenzyl)piperazin-1-yl)- 1 H-indole
[30] 4-(4-benzylpiperazin-1-yl)-1H-indole
[31] 4-(4-(2-fluorobenzyl)piperazin-1-yl)-1H-indole
[32] 4-(4-(naphthalen-2-ylmethyl)piperazin-1-yl)-1H-indole
[33] 4-(4-(2-chlorobenzyl)piperazin-1-yl)-1H-indole
[34] 4-(4-(3,4-dichlorobenzyl)piperazin-1-yl)-1H-indole
[35] 4-(4-(2,4-dichlorobenzyl)piperazin-1-yl)-1H-indole
[36] 4-(4-(3-nitrobenzyl)piperazin-1-yl)-1H-indole
optionally in form of one of its stereoisomers, preferably enantiomers or diasteromers, a racemate or in form of a mixture of at least two of its stereoisomers, preferably enantiomers and/or diasteromers, in any mixing ratio, or a salt thereof, or a corresponding solvate thereof for the manufacture of a medicament for the treatment of a 5-HT₇ mediated disease or condition.

4. Use of a compound according to any of the preceding claims wherein the 5HT₇ mediated disease or condition is pain, sleep disorder, shift worker syndrome, jet lag, depression, seasonal affective disorder, migraine, anxiety, psychosis, schizophrenia, cognition and memory disorders, neuronal degeneration resulting from ischemic events, cardiovascular diseases such as hypertension, irritable bowel syndrome, inflammatory bowel disease, spastic colon or urinary incontinence.

5. A pharmaceutical composition comprising at least one of the compounds defined in claim 1, 2 or 3 a pharmaceutically acceptable salt, isomer, prodrug or solvate thereof, and at least a pharmaceutically acceptable carrier, adjuvant, additive or vehicle.
